# EUROPEAN PATENT APPLICATION

(11) **EP 2 808 037 A1**
(43) Date of publication of application: **03.12.2014**
(21) Application number: 13169416.8
(22) Date of filing: 28.05.2013
(51) Int. Cl.: A61K 49/18

(54) **Paramagnetic beads having a surface charge and method for intracellular magnetic labeling**

(71) Applicant: Miltenyi Biotec GmbH, 51429 Bergisch Gladbach (DE)
(72) Inventor: Dose, Christian, Dr., D-51429 Bergisch Gladbach (DE); Eckardt, Dominik, Dr., 51429 Bergisch Gadbach (DE); Steiner, Mark-Steven, Dr., 51429 Bergisch Gadbach (DE)
(74) Representative: Kisters, Michael Marcus

(57) **Abstract**

The invention relates to paramagnetic beads comprising iron oxide and aminodextran having a zeta potential of 5 to 100 mV.

The paramagnetic beads can be used for in vivo cell tracking by MRI.

## Description

The invention is directed to paramagnetic beads having a certain surface charge expressed by their zeta potential, a method for intracellular magnetic labeling using the paramagnetic beads and a method to produce the paramagnetic beads.

### Prior Art

To separate living cells, it is long known to use paramagnetic particles (beads) functionalized with antibodies on the surface. Cells having the corresponding antigen will bind to the beads and can be separated from cells lacking this antigen by subjecting the cell mixture to a strong magnetic field. The person skilled in the art knows, for example, magnetic cell sorting and the manufacturing of suitable paramagnetic beads from US 5385707. The paramagnetic beads are usually composed of an iron oxide core coated with a dextran shell.

It is furthermore known to use paramagnetic beads for in vivo imaging and tracking of cells. For example WO 2008/144028 discloses the use of dextran-coated magnetic particles for magnetic resonance imaging (MRI) of living cells. The utilized magnetic beads are biocompatible and incorporated into the cells by the aid of a transfection agent. Likewise, various other MRI contrast agents, for example, perfluorocarbon- or iron oxide-based particles, i.e., Cell Sense (CelSense Inc., USA), Endorem (Guerbet Group, France), and Resovist (Bayer Schering Pharma AG, Germany) also show improved cellular uptake of the labeling agent by the use of certain transfection agents. By themselves, many transfection agents are harmful to cells, with the toxic effect being proportional to the transfection agent concentration. Moreover, labeling procedures containing transfection agents can be complex and complicate the downstream processing of the labeled cells.

Especially, for clinical or therapeutically driven applications, it would be desirable to label cells intracellular without the use of transfection agents or other methodologies, e.g. magnetofection or electroporation that rival with cell viability and/or complex labeling protocols.

Surprisingly, it was found that paramagnetic beads comprising iron oxide and aminodextran having a certain surface charge expressed by their zeta potential facilitate the intracellular labeling of various cell types without the aid of additional transfection agents. The cellular uptake of such paramagnetic beads depends on the zeta potential of the paramagnetic beads, while the zeta potential itself relates to the content of positively charged amino groups per aminodextran molecule.

Object of the invention are therefore paramagnetic beads comprising iron oxide and aminodextran having a zeta potential of 5 to 100 mV, preferable 5 to 30 mV.

The zeta potential of the paramagnetic beads according to the invention is determined by dynamic light scattering using for example a Beckman Coulter Delsa Nano HC Particle Analyzer and a standard flow cell with a scattering angle of 15°. Prior to analysis the paramagnetic beads are suspended in distilled water containing 0.1% Pluronic F68 (w/w) and diluted with 0.01 M NaCl in order to adjust the conductivity of the sample to 1.2 ±0.2 mS/cm. The zeta potential of the paramagnetic beads is determined as the mean value from at least six measurements of the same particle lot.

The paramagnetic beads of the invention may consist of an iron oxide core and an aminodextran shell or of a mixture of iron oxide and aminodextran. The paramagnetic beads may also comprise a mixture of aminodextran and dextran (i.e. dextran without amino groups). The ratio of aminodextran and dextran is not particular limited, as long as the resulting beads have the required zeta potential.

Preferable, the aminodextran used to produce the beads of the invention comprise at least 10 amino groups per molecule, preferable 10 to 300 amino groups, and especially 10 to 100 amino groups. The aminodextran and/or the aminodextran/detran mixture have preferable a molecular weight of 30 to 200 kDa.

The term "amino groups" refers to -NH₂, -NHR, and/or NR₂ groups, wherein R depicts a residue having 1 to 100 carbon atoms with and without substitutions for oxygen, nitrogen, and sulfur atoms.

Suitable aminodextran is commercially available from Fina Biosolutions LLS or can be prepared according the method disclosed by Weihong Chen et al. in ACS Appl. Mater. Interfaces, 2011, 3 (10), pp 4085-4091.

The number of amino groups per dextran molecule is determined by the method described by the following publications
1) Habeeb, A.F.S.A. (1966). Determination of free amino groups in protein by trinitrobenzene sulfonic acid. Anal. Biochem. 14, 328;
2) Inman, J.K., Dintzis, H.M. (1969). Biochem. 8, 4074.
3) Sashidhar, R.B., Capoor, A.K., Ramana, D. (1994). Quantitation of ∑-amino group using amino acids as reference standards by trinitrobenzene sulfonic acid. J. Immunol. Meth. 167, 121-127.

The method for determination of the number of amino groups per dextran molecule used in the present invention is based on a chemical reaction of 2,4,6-trinitrobenzenesulfonic acid (TNBSA) with molecules that possess a primary amino group. Briefly, 6-amino-1-hexanol is used as standard reagent for the generation of a calibration plot. The reaction of TNBSA and the aminodextran sample is conducted at 37°C for 2 h at alkaline conditions. Afterwards, the solution is neutralized by the addition of 1 M HCl containing 10% sodium dodecylsulfate (SDS) and the absorbance of the reaction product is monitored at 335 nm. The number of amino groups per molecule dextran is calculated from the calibration slope generated from reactions with defined concentrations of 6-amino-1-hexanol as standard reagent with respect to the determined absorption values.

It is furthermore preferred that the paramagnetic beads possess a mean hydrodynamic diameter of 50 to 5000 nm, more preferably of 75 to 500 nm, and most preferred between 100 and 300 nm.

The hydrodynamic diameter of the paramagnetic beads according to the invention is determined by dynamic light scattering, for example, using a Beckman Coulter Delsa Nano HC Particle Analyzer and a standard disposable plastic cuvette with a scattering angle of 165°. Prior to analysis the paramagnetic beads are suspended in distilled water. The hydrodynamic diameter is calculated using the CONTIN algorithm. The mean value of the intensity weighted size distribution is used as expression of the hydrodynamic diameter of the paramagnetic beads.

In further embodiments of the invention, the paramagnetic beads are provided with at least one marker molecule (reporter molecule) to serve additional imaging modalities. Preferable, the paramagnetic beads of the invention comprise marker molecules that provide a fluorescence and/or luminescence radiation upon excitation in order to serve optical imaging (OI) applications. Such marker molecules, for example, from the classes of coumarines (e.g. VioBlue), xanthines (e.g. FITC), rhodamines (e.g. Texas Red) and/or cyanines (e.g. Cy5.5) are known to the person skilled in the art and can be covalently bound to the beads of the invention via the amino groups of the aminodextran by utilizing common known chemistry.

Furthermore, marker molecule serving imaging modalities like single-photon emission computed tomography (SPECT), x-ray computed tomography (CT), and/or positron emission tomography (PET) might also be conjugated to the paramagnetic beads of the invention. Good examples of multimodality imaging are given, for example, by the rapid evolution of PET-SPECT and PET-CT scanner hybrids as well as growing applications governed by the combination of MRI and OI.

Another object of the invention is a method to produce paramagnetic beads comprising iron oxide and aminodextran having a zeta potential of 5 to 100 mV by providing an aqueous solution of aminodextran and iron salts and subsequently addition of alkaline substances to the solution at a temperature between 30 and 100 °C.

The method comprises first to provide an aqueous solution of iron salts, especially iron salts having chloride, bromide, nitrate, formates, acetate, oxalate, sulfate as anion. The iron salts may contain Fe2+ and /or Fe3+ ions, preferable in a 1:1 to 1:5 mixture based on weight.

To this solution, aminodextran or the aminodextran/detran mixture is added and the resulting solution is heated at a temperature between 30 and 100 °C. At this temperature and under stirring, alkaline substances like sodium hydrogen carbonate, sodium hydroxide, or potassium hydroxide, are added until the desired paramagnetic beads precipitate. The resulting beads are separated from the reaction mixture and washed with water or PBS buffer. The beads can be further purified by enrichment of the paramagnetic beads and/or discharging the not paramagnetic beads by magnetic separation techniques.

Unlike other preparation methods disclosed, for example, by Stefan Miltenyi et al. Cytometry 1990, 11: 231-238, the method according to the invention does not include multiple synthesis steps to achieve aminodextran-coated iron oxide nanoparticles.

The process of the invention provides an easy and robust method for the production of paramagnetic beads comprising aminodextran and iron oxide, which allows the adjustment of the zeta potential of the particles by a one-pot precipitation process. The approach requires the addition of respective aminodextran molecules to the single-step synthesis and does not involve multiple synthesis steps to furnish positively charged paramagnetic beads.

### Intracellular labeling of cells

Another object of the invention is the use of the paramagnetic beads according to the invention for intracellular labeling of cells. Labeled cells can be detected or monitored by MRI.

If the paramagnetic beads according to the invention comprise additional marker molecules, the labeled cells can be detected or monitored with the aid of other imaging modalities, for example, by optical imaging methods collecting a fluorescence or luminescence signal, by single-photon emission computed tomography (SPECT), x-ray computed tomography (CT), and/or positron emission tomography (PET)

The paramagnetic beads of the invention can be introduced into cells *in vitro* by standard co-culturing procedures. It is beneficial for uptake of the beads either to use adherent cells or to plate cells on cell culture dishes in culture medium. No further additive in the culture medium is needed to allow for uptake of the paramagnetic beads. For example, respective cells are cultivated for 4 h at 37 °C in the presence of the paramagnetic beads. After the incubation period, the cells are washed with PBS buffer to remove the labeling solution, harvested by standard procedures, and ready to be utilized for downstream applications.

The paramagnetic beads of the invention are especially useful for labeling of mammalian cell types useable in cell therapeutic applications like adult stem cells, e.g. mesenchymal stem cells, hematopoietic stem cells, cardiac stem cells, like pluripotent stem cells and pluripotent stem cell-derived cells, immune cells, like T-cells, dendritic cells, monocytes and macrophages as well as cancer cells. The paramagnetic beads of the invention are furthermore useful for labeling of bacteria or plant cells.

### Cell tracking by magnet resonance imaging

Another object of the invention is a method for in vivo cell tracking wherein cells are intracellularly labeled with paramagnetic beads according to the invention, the labeled cells introduced into a living organism and detected by MRI. If the paramagnetic beads according to the invention comprise additional marker molecules, the labeled cells can be detected or monitored in the living organism with the aid of additional marker molecules, for example, by fluorescence or luminescence detection. Of course, both methods of detection i.e. MRI in combination with the signal produced by the additional marker molecules can be used.

In the method of the invention, the paramagnetic beads may be introduced in the cells ex vivo and the labeled cells are then introduced into the living organism to detect the labeled cells in vivo. It is furthermore possible to introduce the paramagnetic beads directly into the living organism, where the beads are taken up by the cells in vivo. Again, the labeled cells are then detected in vivo.

Magnet resonance imaging (MRI) is the most frequently used technique for serial *in vivo* cell tracking applications due to high resolution of soft tissues. In order to improve detectability of transplanted cells and produce a strong contrast against surrounding tissue, intracellular labeling of cells with iron oxide particles before transplantation and the respective tracking of the cells by MRI has been described, for example, by Hoehn, M. et al. (2007) J. Physiol. 584: 25-30).

The paramagnetic beads of the invention are especially useful to track the movement or distribution of labeled cells in animals or in the human body. In another application, the paramagnetic beads can be used for *in vivo* detection of tumors and inflammatory sites by passive targeting of phagocytic cells. These cells take up foreign particles from the body thereby accumulating paramagnetic beads intracellularly and deliver a more intense MRI signal than the surrounding tissue.

### Examples

### Synthesis of paramagnetic beads according to the invention

15.5 g of aminodextran with an average molar mass of 40 kDa having 25 amino groups per molecule is dissolved in 14.5 g of distilled water and stirred over night. The aminodextran solution is degassed at low pressure for 60 min to give a clear yellowish viscous liquid. 1.87 g of FeCl₃•6 H₂O and 0.8 g of FeCl₂•4 H₂O are dissolved in 8.3 mL of distilled and degassed water and added under vigorous stirring to the prepared the aminodextran solution. The orange colored solution is heated to 35°C while stirring. Subsequently, 12 mL of a 4 M NaOH are added to the solution using a syringe pump with a rate of 0,2 mL/min. The solution turns brownish to black during the reaction. The temperature of the mixture is set to 70 °C for 5 min and then cooled down to room temperature. Finally, 37.4 mL of distilled H₂O are added and the particle solution is stored over night at 4 °C.

### Work up of the paramagnetic beads according to the invention

The volume of the solution is made up to 200 mL. The particles are centrifuged at 300 rpm for 15 min to remove large aggregates. A D-column is mounted to a SuperMACS (Miltenyi Biotec), filled with 60 mL of 70% ethanol and consecutively washed with 600 mL of distilled H₂O and 600 mL of 10 mM PBS buffer containing 5 mM EDTA. The particle solution is subsequently filled onto the column using a peristaltic pump with a dispense rate of 8 mL/min. The particles are washed on the column with 300 mL of PBS-EDTA buffer and the nanoparticles that are not retained in the magnetic field are discarded. The column is removed from the magnet and the particles are eluted with 100 mL of PBS-EDTA buffer using a dispense rate of 25 mL/min.

### Modification of the particles using a fluorescent dye

80 mg of the particles according to Ex2 are transferred in PBS to 0.4 M borate buffer (pH 9). Then, 38.5 nmol of the amine reactive fluorescent dye (e. g. fluorescein isothiocyanate in Ex3 and NHS-VioBlue in Ex4) per 1 mg of particles are added. The mixture is shaken for 60 min at room temperature. Finally, excess dye is removed by washing the labeled particles with an appropriate amount of 0.04 M borate buffer.

### Determination of the hydrodynamic diameter

The hydrodynamic diameter of the paramagnetic beads according to the invention is determined using a Beckman Coulter Delsa Nano HC Particle Analyzer and standard disposable plastic cuvettes with a scattering angle of 165°. The paramagnetic beads are suspended in water. The hydrodynamic diameter is calculated using the CONTIN algorithm. The mean of the intensity weighted size distribution is used as expression of the hydrodynamic diameter of the paramagnetic beads.

### Determination of the zeta potential

The zeta potential of the paramagnetic beads is determined using a Beckman Coulter Delsa Nano HC Particle Analyzer and the standard flow cell with a scattering angle of 15°. The particles are transferred to plain water containing 0.1% Pluronic F68 (w/w) and diluted with 0.01 M NaCl to yield a conductivity of the sample of 1.2 ±0.2 mS/cm. The zeta potential was then evaluated from at least 6 measurements.

Table 1 shows the chemical and physical features of paramagnetic beads according to the invention (Ex1 - Ex4) in comparison with paramagnetic beads not according to the invention (C1). All beads were prepared according to the above described synthesis method, using aminodextran with different amount of amino groups or by using dextran instead of amino dextran (C1).

### Intracellular magnetic labeling of cells

NIH 3T3 cells were plated on cell culture dishes in culture medium before intracellular labeling. The paramagnetic beads of the examples Ex1 and Ex 2 and the comparison example C1 were added to culture dishes at optimized concentrations for this cell type, e. g., 100 µg per 1x10E6 NIH-3T3 cells. After the incubation period, the cells are washed with PBS buffer to remove the labeling solution, harvested by standard procedures, and stained with Prussian Blue to detect the uptake of the beads.

The figures show:

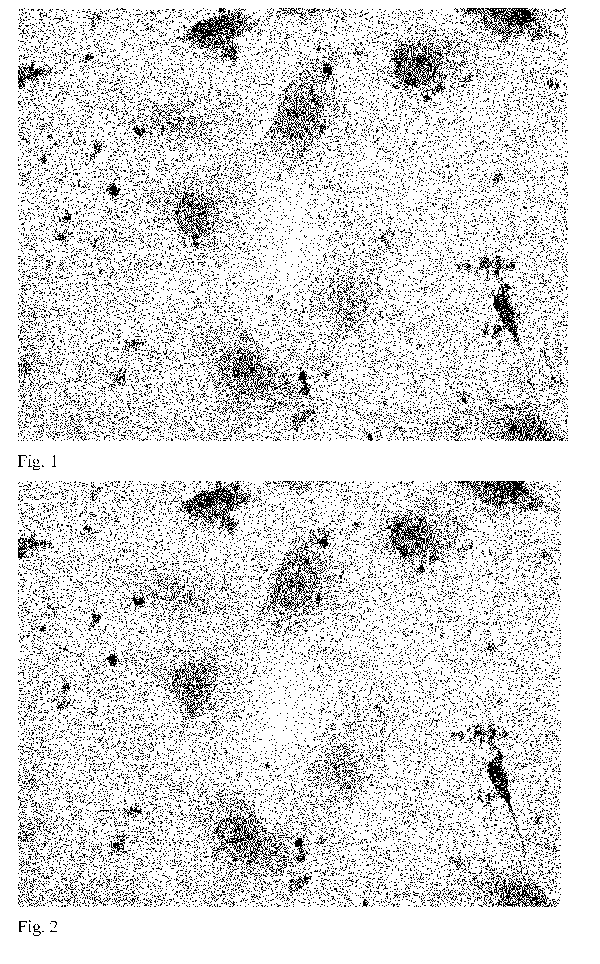

- Fig. 1:: NIH 3T3 cells labeled with paramagnetic particles according to the comparison example i.e. having a zeta potential of <0 mV. Iron oxide particles are detected by Prussian Blue staining, but no perinuclear staining is visible.
- Fig. 2:: Mesenchymal stem cells labeled with paramagnetic particles according to the comparison example i.e. having a zeta potential of <0 mV. Iron oxide particles are detected by Prussian Blue staining, but no perinuclear staining is visible.

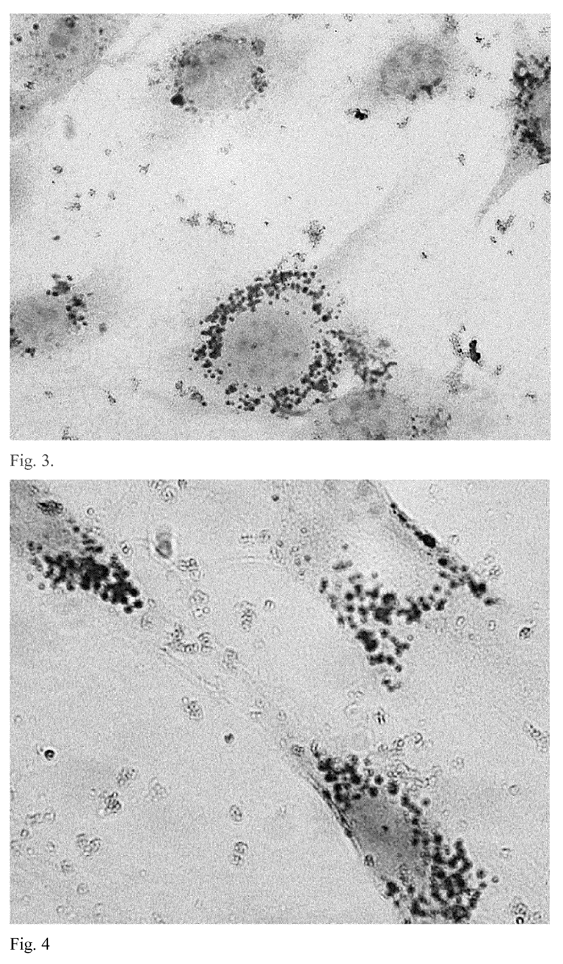

- Fig. 3:: NIH 3T3 cells labeled with paramagnetic beads according to the Ex 2. Iron oxide particles are detected by Prussian Blue staining. Strong perinuclear staining visible.
- Fig. 4:: Mesenchymal stem cells labeled with paramagnetic beads according to the Example 2. Beads are detected by Prussian Blue staining. Strong perinuclear staining visible.

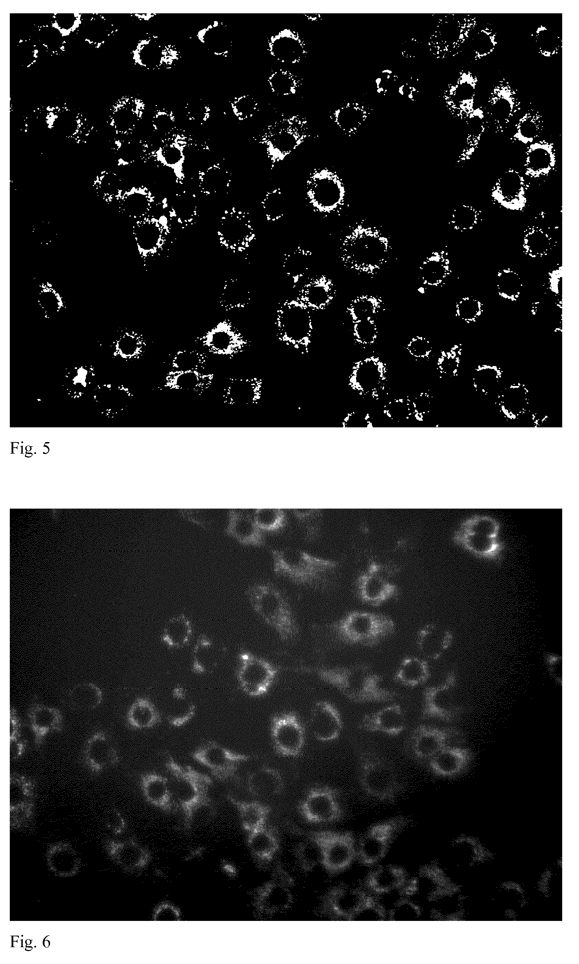

- Fig. 5:: NIH 3T3 cells labeled with FITC modified paramagnetic beads according to Ex 3 and to the cell labeling protocol. Fluorescence of FITC is located mainly perinuclear.
- Fig. 6:: NIH 3T3 cells labeled with VioBlue modified paramagnetic beads according to Ex 4 and to the cell labeling protocol. Fluorescence of VioBlue is located mainly perinuclear.

**Table 1**

| Example | Dextran | hydrodynamic diameter (nm) | marker molecule | CV (nm) | polydispersity index | zeta potential (mV) | iron content (pmol/mg) | amino groups per molecule dextran |
|---|---|---|---|---|---|---|---|---|
| C1 | 100% dextran | 107 | - | 35 | 0.110 | -3.11 | 6.4 | 0 |
| Ex1 | 100% aminodextran | 114 | - | 48 | 0.103 | 8.23 | 5.3 | 27 |
| Ex2 | 100% aminodextran | 155 | - | 50 | 0.126 | 16.1 | 7.1 | 45 |
| Ex3 | 100% aminodextran | 155 | FITC | 50 | 0.126 | 14.8 | 7.1 | 45 |
| Ex4 | 100% aminodextran | 155 | VioBlue | 50 | 0.126 | 15.0 | 7.1 | 45 |

## Claims

1. Paramagnetic beads comprising iron oxide and aminodextran **characterized by** a zeta potential of 5 to 100 mV.

2. Paramagnetic beads according to claim 1 **characterized by** comprising a mixture of aminodextran and dextran.

3. Paramagnetic beads according to claim 1 **characterized in that** the aminodextran comprises at least 10 amino groups per molecule.

4. Paramagnetic beads according to claim 1 **characterized in** a mean hydrodynamic diameter of 50 to 5000 nm.

5. Paramagnetic beads according to claim 1, **characterized by** comprising a marker molecule.

6. Method to produce paramagnetic beads comprising iron oxide and aminodextran having a zeta potential of 5 to 100 mV by providing an aqueous solution of aminodextran and iron salts and subsequently add alkaline substances to the solution at a temperature of 30-100 °C.

7. Use of the paramagnetic beads according to claim 1 for intracellular labeling of cells.

8. Use according to claim 7 **wherein** the intracellularly labeled cells are detected by MRI.

9. Use according to claim 7 **wherein** the intracellular labeled cells are detected by fluorescence, luminescence, single-photon emission computed tomography (SPECT), x-ray computed tomography (CT), and/or positron emission tomography (PET).

10. Method for in vivo cell tracking **characterized in that** cells are intracellularly labeled with paramagnetic beads according to claim 1, the labeled cells introduced into a living organism and detected by MRI.

11. Method for in vivo cell tracking **characterized in that** cells are intracellularly labeled with paramagnetic beads according to claim 1, the labeled cells introduced into a living organism and detected by fluorescence, luminescence, single-photon emission computed tomography (SPECT), x-ray computed tomography (CT), and/or positron emission tomography (PET).
